# EUROPEAN PATENT APPLICATION

(11) **EP 2 497 510 A1**
(43) Date of publication of application: **12.09.2012**
(21) Application number: 10828341.7
(22) Date of filing: 05.11.2010
(51) Int. Cl.: A61M 5/32, A61M 5/28

(54) **SYRINGE-CUM-CONTAINER**

(30) Priority: 05.11.2009 JP 2009254331
(71) Applicant: ARTE CORPORATION, Shiyoda-ku Tokyo 101-0032 (JP)
(72) Inventor: KAKIUCHI Makoto, Takahagi-shi Ibaraki 318-0004 (JP)
(74) Representative: De Vries & Metman
(86) International application number: PCT/JP2010/069688
(87) International publication number: WO 2011/055782

(57) **Abstract**

In a container-cum-syringe (1) including a safety device (50), a coil spring (59) which biases the safety device (50) forward, first holding means (3) for causing the safety device (50) to be in a fastened state at a retreated position against the biasing of the coil spring (59), fastening releasing means (80) which is provided slidably along a radial direction of an axis (O) for releasing the fastened state of the safety device (50) by the first holding means (3) through a sliding movement, and locking means (70) for locking the fastened state of the safety device (50) by the first holding means (3) are provided. In this configuration, the safety device may be easily moved to a position for covering the injection needle. In addition, a malfunction of the safety device during an injecting operation may be reliably prevented.

## Description

### Technical Field

The present invention relates to a combined container-syringe including a safety device used for ensuring safety by covering an injection needle after an administration of injection or the like.
Priority is claimed on Japanese Patent Application No. 2009-254331, filed on November 5, 2009, the content of which is incorporated herein by reference.

### Background Art

Since a combined container-syringe is pre-filled with a medical solution, it can be used immediately after being removed from the packaging without performing a complicated operation at a medical institution. Therefore, it has outstanding convenience and is extremely useful in lightening the workload of doctors and nurses, and for this reason has been adopted in many hospitals and clinics.

However, hitherto, when a syringe is used in the medical practice, there may be cases where the medical personnel pierce themselves after puncturing with the injection needle by mistake, and there is concern of the transmission of viruses caused by the puncturing. Therefore, for the purposes of safely processing the injection needle after injecting a patient, a syringe has been designed which includes a safety device which prevents inadvertent contact of the injection needle by covering the injection needle after injection with a cylindrical cover.

For example, in a syringe described in Patent Document 1, a pressure is applied to a flexible safety device fastend to the outer periphery of the cylinder so as to be bent, thereby releasing the fastened state. In addition, as the injection needle is directed upward in a state of interposing the safety device, a main body of the syringe is moved downward by its own weight, such that the injection needle is covered by the safety device.

### Citation List

### Patent Document

[Patent Document1 Japanese Patent Application, First Publication No. 2001-29334

### Summary of Invention

### Technical Problem

However, in the syringe including the conventional safety device as described above, only the weight of the main body of the syringe is means for moving the safety device to a position for covering the injection needle. Therefore, a force to relatively move the safety device is weak, and under some circumstances, the injection needle may be caught on the main body of the syringe while being covered by the safety device. As a result, there may be cases where the injection needle may not be reliably covered.

In addition, manually moving the safety device may be considered. However, in this case, while the combined container-syringe itself is supported in one hand, the other hand needs to hold and move the safety device. Therefore, operations with both hands are needed, and thus the operation becomes cumbersome.

In addition, in the syringe including the conventional safety device as described above, the safety device is only easily attached to an engagement portion by friction. Therefore, the safety device is not firmly fixed, and depending on the case, there is a possibility of the safety device deviating from the syringe and exposes the injection needle.
Moreover, during an operation of performing an injection for a patient, it is preferable that the safety device be reliably held at a predetermined position so as not to cause the safety device to malfunction.

In order to solve the problems, an object of the invention is to provide a combined container-syringe capable of easily moving a safety device to a position where the injection needle is covered, and reliably preventing a malfunction of the safety device during an injection operation, thereby ensuring safety.

### Solution to Problem

In order to accomplish the object, the invention suggests the following means.
A combined container-syringe according to the invention includes: a main body of combined container-syringe in which a cylindrical tip to which an injection needle is mounted is attached to a front end side of an outer cylinder that is filled with a medical solution and extends along an axis, and a finger grip is attached to a rear end side of the outer cylinder; a safety device which is slidably attached to an outer peripheral side of the outer cylinder, and covers the injection needle when being moved forward; biasing means for biasing the safety device forward; biasing means for biasing the safety device forward; holding means for causing the safety device to be in an fastened state at a retreated position against biasing of the biasing means; fastening releasing means which is provided slidably along a radial direction around the axis and releases the fastened state of the safety device by the holding means according to a sliding movement; and locking means for locking the fastened state of the safety device by the holding means.

According to the combined container-syringe, the fastened state of the safety device by the holding means is released by moving the fastening releasing means in the radial direction. Accordingly, the safety device is moved forward by the biasing of the biasing means and covers the injection needle. That is, only by the operation of moving the holding means to slide is the safety device moved forward, so that the injection needle may be covered by the safety device easily and reliably. In addition, since the fastened state of the safety device by the holding means is locked by the locking means, for example, even in a case where an external force is abruptly exerted to release the fastened state by the holding means, the fastened state is not released.

In addition, in the combined container-syringe according to the invention, it is preferable that the fastening releasing means be configured to, during the sliding movement from a standby position positioned on an outside in the radial direction to a release position positioned on an inside in the radial direction, first, release the locking by the locking means, and second, release the fastening by the fastening releasing means.

According to the combined container-syringe, by a single sliding movement of the fastening releasing means, the locking by the locking means and the fastened state by the fastening releasing means may be released. Therefore, the safety device may be operated with an easy operation while reliably preventing a malfunction of the safety device.

Moreover, in the combined container-syringe according to the invention, it is preferable that the holding means include a fastening portion provided in the main body of combined container-syringe, and an engagement portion that is provided at a rear end portion of the safety device and is engaged with the fastening portion from the outside in the radial direction, and the locking means have a pressing portion that presses the engagement portion from the outside in the radial direction.

According to the combined container-syringe, as the engagement portion on the safety device side is engaged with the fastening portion on the combined container-syringe side from the outside in the radial direction, the safety device may be reliably in the fastened state at the retreated position. In addition, as the pressing portion of the locking means further presses the engagement portion from the outside in the radial direction, the fastened state by the holding means may be reliably locked.

In addition, in the combined container-syringe according to the invention, it is preferable that a rib on which the pressing portion abuts be provided on a surface on the outside in the radial direction of the engagement portion.
Accordingly, when the pressing portion presses the engagement portion, contact pressure between the pressing portion and the engagement portion is increased, so that locking of the holding means by the pressing portion may be more reliably performed.

In addition, in the combined container-syringe according to the invention, the locking means may be provided rotatably with respect to the main body of combined container-syringe, and the fastening releasing means may abut on the locking means when the fastening releasing means is slid, and as the locking means is rotated, the locking by the locking means may be released.
On the other hand, the locking means may be provided slidably along the radial direction, and as the locking means is slid as the fastening releasing means is slid, the locking by the locking means may be released.
Accordingly, when the fastening releasing means is slid, the locking by the locking means may be released.

Moreover, in the combined container-syringe according to the invention, it is preferable that, in the finger grip, a finger contact cover that surrounds an outer peripheral portion of a rear end of the safety device at the retreated position from an outer peripheral side be provided.

This is an improvement for eliminating the possibility of, in a case where the finger of a doctor, a nurse, or the like comes into contact with the outer peripheral portion of the rear end of the safety device when the locking by the locking means and the fastened state by the holding means released, forward movement of the safety device by the biasing of the coil spring may be impeded, and the safety device may not be normally operated.
That is, according to the combined container-syringe having the above-described characteristics, since the finger contact cover which surrounds and covers the outer peripheral portion of the rear end of the safety device from the outer peripheral side is provided, the finger of the doctor, nurse, or the like comes into contact with the finger contact cover and does not come into contact with the safety device. As a result, the operation of the safety device is not impeded, and the injection needle may be reliably covered by the safety device.

### Advantageous Effects of Invention

According to the combined container-syringe of the invention, only by operating the fastening releasing means may the safety device be moved to a position for reliably covering the injection needle. Therefore, the safety device may be easily and reliably moved to the position where the safety device covers the injection needle, so that safety may be ensured.
In addition, since the fastening releasing means is locked by the locking means, a malfunction of the safety device is reliably prevented, and safety may be ensured.

### Brief Description of Drawings

FIG. 1 is a side view of a part of a combined container-syringe according to a first embodiment of the invention in cross-section.
FIG. 2 is a partial enlarged view of FIG. 1.
FIG. 3 is a diagram of the combined container-syringe according to the first embodiment of the invention as viewed from the rear end side.
FIG. 4 is a side view illustrating a state where a finger grip, fastening releasing means, and locking means according to the first embodiment of the invention are disassembled.
FIG. 5A is a side view of the finger grip according to the first embodiment of the invention.
FIG. 5B is a diagram illustrating the finger grip according to the first embodiment of the invention as viewed from the rear end side.
FIG. 5C is an A-A cross-sectional view of the finger grip according to the first embodiment of the invention in FIG. 5B.
FIG. 6A is a side cross-sectional view of the fastening releasing means according to the first embodiment of the invention.
FIB. 6B is a diagram of the fastening releasing means according to the first embodiment of the invention as viewed from the rear end side.
FIG. 7A is a side view of the locking means according to the first embodiment of the invention.
FIG. 7B is a diagram of the locking means according to the first embodiment of the invention as viewed from the rear end side.
FIG. 7C is a diagram of the locking means according to the first embodiment of the invention as viewed in the direction B in FIG. 7B.
FIG. 8 is a diagram illustrating a state where the fastening releasing means in FIG. 2 is slid.
FIG. 9 is a side cross-sectional view of the rear end side part of a combined container-syringe according to a second embodiment of the invention.
FIG. 10 is a diagram of the combined container-syringe according to the second embodiment of the invention as viewed from the rear end side.
FIG. 11 is a side view illustrating a state where a finger grip, fastening releasing means, and locking means according to the second embodiment of the invention are disassembled.
FIG. 12A is a side view of the finger grip according to the second embodiment of the invention.
FIG. 12B is a diagram of the finger grip according to the second embodiment of the invention as viewed from the rear end side.
FIG. 13A is a diagram of the fastening releasing means according to the second embodiment of the invention as viewed from the rear end side.
FIG. 13B is a side cross-sectional view of the fastening releasing means according to the second embodiment of the invention.
FIG. 14A is a side view of the locking means according to the second embodiment of the invention.
FIG. 14B is a diagram of the locking means according to the second embodiment of the invention as viewed from the rear end side.
FIG. 15 is a diagram illustrating a state where the fastening releasing means in FIG. 10 is slid.
FIG. 16 is a side cross-sectional view of the rear end side part of a combined container-syringe according to a third embodiment of the invention.
FIG. 17 is a diagram of the combined container-syringe according to the third embodiment of the invention as viewed from the rear end side.
FIG. 18A is a side view of a finger grip according to the third embodiment of the invention.
FIG. 18B is a diagram of the finger grip according to the third embodiment of the invention as viewed from the rear end side.
FIG. 19A is a diagram of fastening releasing means according to the third embodiment of the invention as viewed from the rear end side.
FIG. 19B is a side cross-sectional view of the fastening releasing means according to the third embodiment of the invention.
FIG. 20A is a side view of locking means according to the third embodiment of the invention.
FIG. 20B is a diagram of the locking means according to the third embodiment of the invention as viewed from the rear end side.
FIG. 20C is a diagram of the locking means according to the third embodiment of the invention as viewed in the direction B in FIG. 20B.
FIG. 21 is a diagram illustrating a state where the fastening releasing means in FIG. 16 is slid.
FIG. 22 is a side view of a part of a combined container-syringe according to a fourth embodiment of the invention before the operation of the safety device in cross-section.
FIG. 23 is a side view of the part of the combined container-syringe according to the fourth embodiment of the invention after the operation of the safety device in the cross-section.

### Description of Embodiments

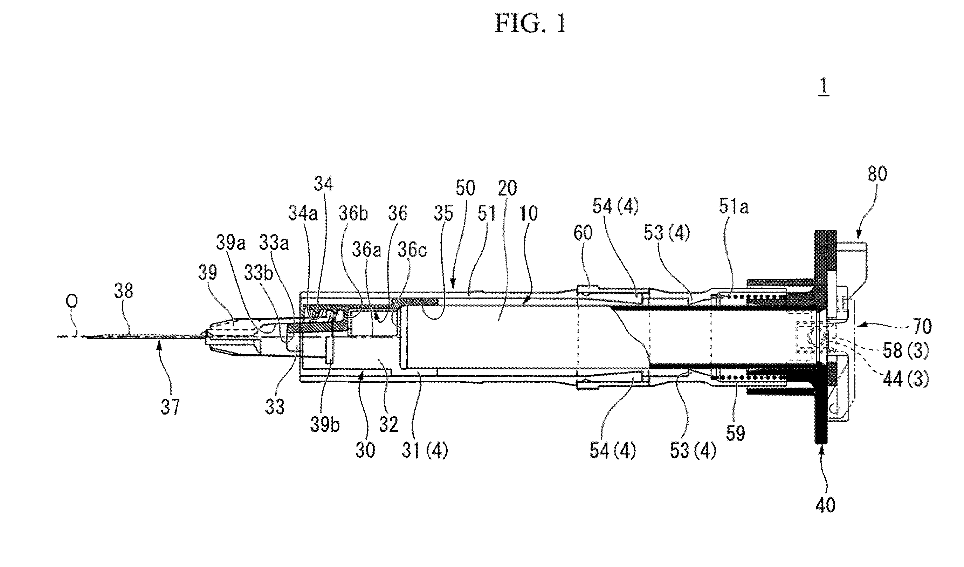

Hereinafter, a first embodiment of a combined container-syringe according to the invention will be described in detail with reference to the drawings. As illustrated in FIG. 1, a combined container-syringe 1 according to the first embodiment is configured as a safety device 50 is mounted to a main body of combined container-syringe 10 extending along an axis O.

The combined container-syringe 1 includes first holding means (holding means) 3 for releasably fastening the safety device 50 to a retreated position deviating from an injection needle 37, second holding means 4 for fastening the safety device 50 to an advanced position for covering the injection needle 37 of the main body of combined container-syringe 10, a coil spring (biasing means) 59 for biasing the safety device 50 from the retreated position toward the advanced position, fastening releasing means 80 for releasing the fastened state of the safety device 50 by the first holding means 3, and locking means 70 for locking the fastened state of the safety device 50 by the first holding means 3.
In addition, the radial direction of the main body of combined container-syringe 10, the tip end side thereof, and the rear end side thereof are simply referred to as the radial direction, the front side, and the rear side, respectively.

As illustrated in FIG. 1, the main body of combined container-syringe 10 is composed of an outer cylinder 20, a hub luer-lock (cylindrical tip) 30 which is attached to the front end side of the outer cylinder 20 and having a tip end to which the injection needle 37 is mounted, and a finger grip 40 attached to the rear end side of the outer cylinder 20.

The outer cylinder 20 is made of a transparent member such as glass or a synthetic resin and has a cylindrical shape extending along the axis O. On the front side of the inside of the outer cylinder 20, a front stopper (not illustrated) is liquid-tightly provided, and on the rear side thereof, a middle stopper (not illustrated) and a rear stopper (not illustrated) are air-tightly provided at an interval along a direction of the axis O. In addition, between the front stopper and the middle stopper and between the middle stopper and the rear stopper, medicine and medical solution are respectively sealed.

The hub luer-lock 30 has an outer multi-step columnar shape with respect to the axis O as the center, which is made of a transparent synthetic resin with appropriate rigidity, and includes a safety device engagement portion 31 that has a cylindrical shape and is positioned on the base end side, a cylindrical portion 32 that is joined to the tip end side of the safety device engagement portion 31 so as to have a diameter reduced in one step, a luer tip 33 that is formed with a diameter smaller than that of the cylindrical portion 32, is joined to the tip end side of the cylindrical portion 32, and extends toward the tip end side along the axis O, a luer lock portion 34 that is joined to the tip end side of the cylindrical portion 32 at an interval on the outer side in the radial direction of the luer tip 33 and has a cylindrical shape with respect to the axis O as the center.

On the inner side of the safety device engagement portion 31, a fitting hole 35 which is open to the rear side and in which the outer periphery of the front end of the outer cylinder 20 is fitted. In addition, on the front side of the fitting hole 35 of the safety device engagement portion 31, that is, inside the cylindrical portion 32, a bypass chamber 36 having a hole shape with a bottom is formed. The bypass chamber 36 is a hole with a bottom of which the inside diameter is smaller than that of the fitting hole 35, and on the inner peripheral wall thereof, a plurality of bypass grooves 36a extending in parallel to the axis O are formed at equal intervals in the peripheral direction. The bypass grooves 36 each extend to a bottom portion 36a of the bypass chamber 36. In addition, the rear end of the bypass groove 36a is connected to an annular groove 36c formed with respect to the axis O as the center at the boundary between the bypass chamber 36 and the fitting hole 35.

The outer peripheral surface of the luer tip 33 is a tapered outer peripheral surface 33a of which the diameter is reduced toward the front side. In addition, in the luer tip 33, a communication hole 33b which penetrates through the inside of the luer tip 33 in the forward and rearward direction along the axis O and communicates with the center of a bottom portion 36b of the bypass chamber 36 is bored.
Moreover, on the inner peripheral surface of the luer lock portion 34, a lock screw 34a for fastening the needle base of the injection needle 37 is formed.

The injection needle 37 is composed of a main body of injection needle 38 extending forward along the axis O and a needle base 39 positioned on the base end side of the main body of injection needle 38. The needle base 39 includes a tapered hole 39a of which the diameter is gradually reduced with respect to the axis O as the center as it goes forward from the rear end portion, and moreover, includes a screwing protrusion 39b protruding toward the outer peripheral side from the rear end. The tapered hole 39a of the needle base 39 is fitted to the tapered outer peripheral surface 33a of the luer tip 33, and the screwing protrusion 39b is screwed to the lock screw 34a of the luer lock portion 34, such that the injection needle 37 is firmly fixed to the front end of the luer lock portion 34.

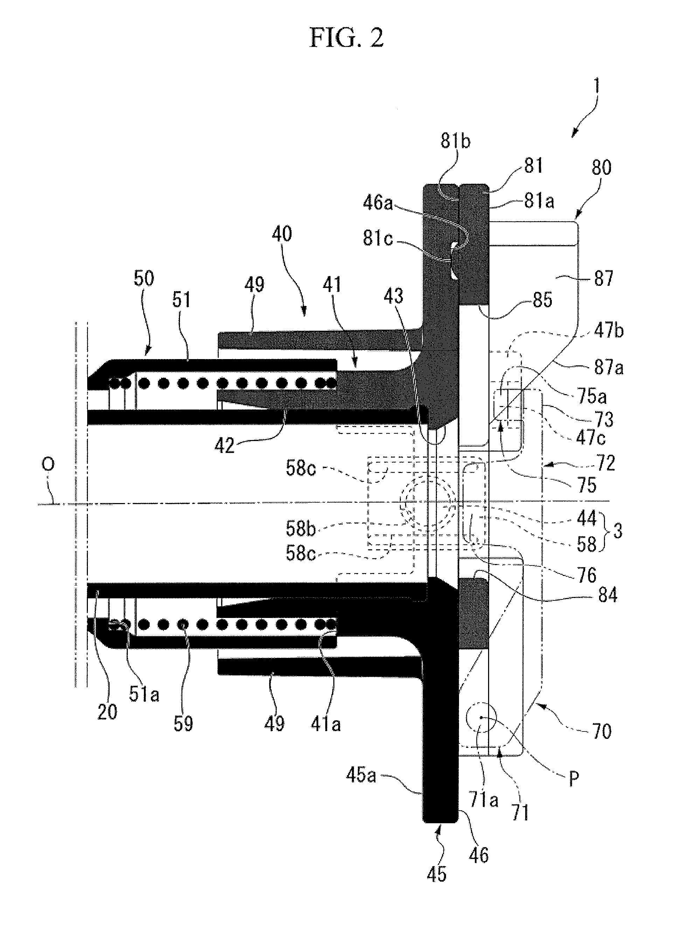

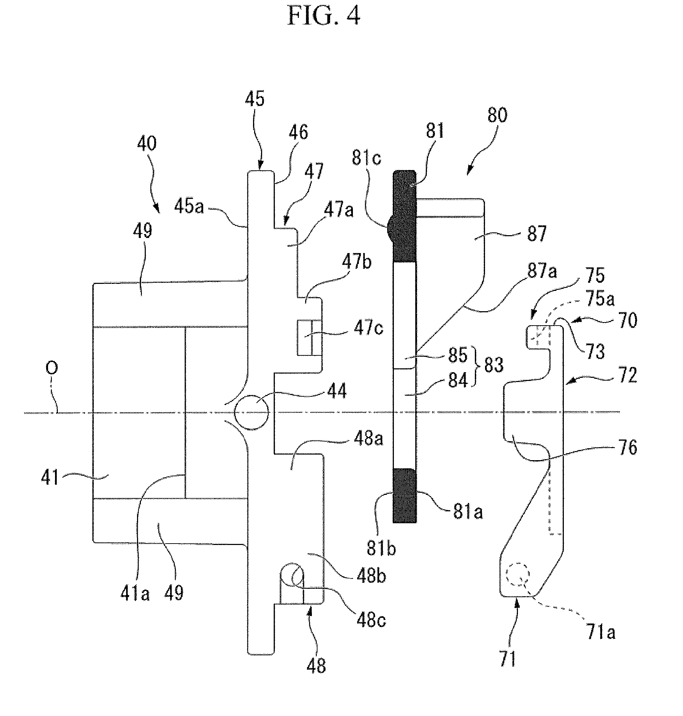

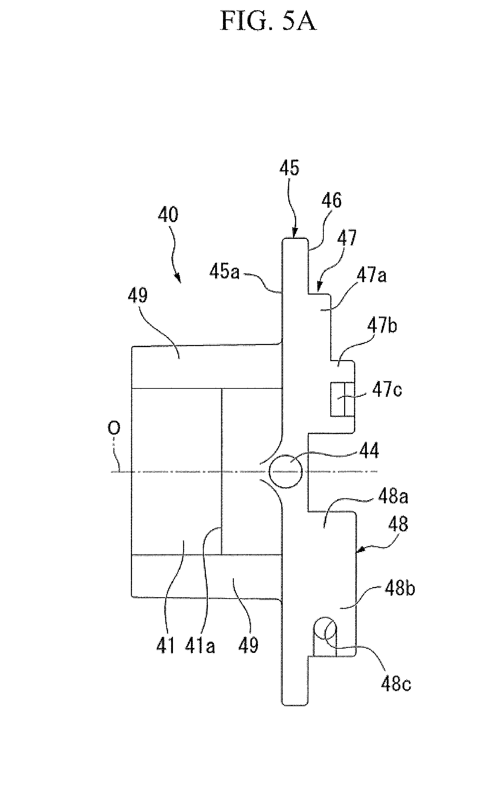

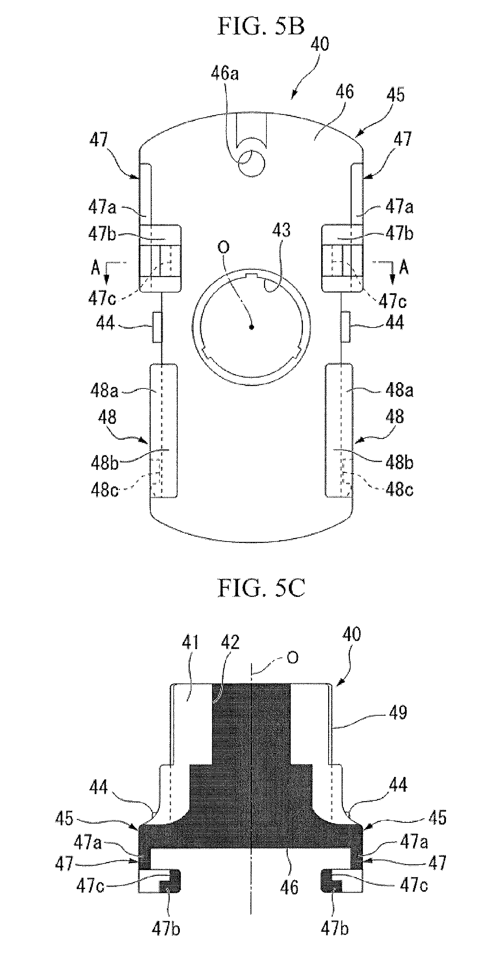

As illustrated in FIGS. 2 to 5C, the finger grip 40 is a member to which the fastening releasing means 80 and the locking means 70 are mounted, and includes a fitting portion 41 having a cylindrical shape and a flange portion 45 extending outward in the radial direction from the rear end portion of the fitting portion 41. Moreover, in this embodiment, a pair of finger contact covers 49 and 49 is provided on the outer peripheral side of the fitting portion 41 at a predetermined interval from the fitting portion 41 in the radial direction. Hereinafter, the structure of the finger grip 40 will be described with reference to FIGS. 5A to 5C.

The inner peripheral side of the fitting portion 41 is a fitting hole 42 into which the rear end portion of the outer cylinder 20 is fitted, and on the rear end side of the fitting portion 41, an insertion hole 43 that is open to the rear side and has a diameter smaller than the fitting portion is formed. In addition, a part on the tip end side in the outer peripheral surface of the fitting portion 41 has a diameter reduced compared to other parts of the outer peripheral surface, and the step portion formed by the diameter reduction so as to be directed in the forward direction is a spring abutting step portion 41a on which the rear end of the coil spring 59 abuts. Moreover, on the outer peripheral portion of the rear end of the fitting portion 41, a pair of fastening convex portions (fastening portions) 44 and 44 are formed which protrude outward in the radial direction in substantially columnar shapes at positions opposing each other with the axis O interposed therebetween at an interval of 180° in the peripheral direction.

As illustrated in FIG. 5B, the flange portion 45 has a substantially rectangular shape that is relatively short in a direction in which the fastening convex portions 44 and 44 oppose each other (hereinafter, referred to as a width direction) and has a direction orthogonal to this direction as a longitudinal direction. A surface directed rearward in the flange portion 45 is a flange surface 46 having a flat shape, and the insertion hole 43 that is circular is open at the center of the flange surface 46. In addition, a surface directed forward in the flange portion 45 is a finger hooking surface 45a.
In addition, at an end portion on one side in the longitudinal direction of the flange surface 46, a fastening concave portion 46a which is dent in a spherical surface shape is formed.

At both end portions of the width direction of the flange portion 45, a pair of first guide portions 47 and 47 and a pair of second guide portions 48 and 48 are respectively provided to oppose along the width direction.

The first guide portions 47 and 47 are provided at both end portions of one side in the longitudinal direction of the flange portion 45. The first guide portions 47 and 47 are composed of side guides 47a and 47a that are erected vertically from the flange surface 46 in the rearward direction and extend along the longitudinal direction, and pressing guides 47b and 47b that extend from parts close to the center in the longitudinal direction at the upper edges of the side guides 47a and 47a so as to approach each other.

In addition, on the surface on the opposite side to the side where the first guide portions 47 and 47 oppose each other, rectangular grooves 47c and 47c for fixing the locking means 70 to the flange portion 45 are formed.

The second guide portions 48 and 48 are provided at both end portions on the other side in the longitudinal direction of the flange portion 45. The second guide portions 48 and 48 are composed of side guides 48a and 48a that are erected vertically from the flange surface 46 in the rearward direction and extend along the longitudinal direction, and pressing guides 48b and 48b that extend from the upper edges of the side guides 48a and 48a so as to approach each other, and have substantially L shapes in cross-section. In addition, the distance between the pair of the second guide portions 48 and 48 is shorter than the distance between the pair of the first guide portions 47 and 47.

In addition, on the surface on the opposite side to the side where the second guide portions 48 and 48 oppose each other, attaching holes 48c and 48c for rotatably attaching the locking means 70 are formed respectively. In addition, the attaching holes 48c and 48c are formed at positions separated from the center in the longitudinal direction of the flange portion 45.

The finger contact covers 49 form a pair so as to oppose each other with respect to the axis O as the center. The finger contact covers 49 extend forward from the finger hooking surface 45a of the flange portion 45 and have arc shapes with respect to the axis as the center in a cross-section orthogonal to the axis O. The pair of the finger contact covers 49 and 49 are disposed at a predetermined distance from the outer peripheral surface of the fitting portion 41, and accordingly, gaps are formed between the finger contact covers 49 and 49 and the outer peripheral surface of the fitting portion 41.

To the outer periphery of the main body of combined container-syringe 10 composed of these members, the safety device 50 is mounted. The safety device 50 is composed of, as illustrated in FIG. 1, the safety device body 51 and a pressing ring 60.

The safety device body 51 is formed of a material that is transparent and has an appropriate flexibility, for example, polypropylene, so as to be thin in a range capable of maintaining the strength, and has a substantially cylindrical shape with respect to the axis O as the center. In addition, the inside diameter of the safety device body 51 is formed to be substantially equal to the outside diameter of the outer peripheral surface of the safety device engagement portion 31.

On the inner peripheral surface of the safety device body 51, a plurality of slip-out prevention stoppers 53 which protrude inward in the radial direction and are inclined inward in the radial direction as they go to the front side are formed at equal intervals in the peripheral direction.

Moreover, in front of the slip-out prevention stopper 53, a plurality of return prevention stoppers 54 which protrude inward in the radial direction from the inner peripheral surface by being pressed inward in the radial direction by the pressing ring 60 are formed at equal intervals in the peripheral direction. The return prevention stoppers 54 are inclined so as to protrude inward in the radial direction as they go to the rear side.

The pressing ring 60 mounted to the safety device body 51 has a cylindrical shape in which the length thereof in the direction of the axis O is sufficiently shorter than that of the safety device body 51, and is formed of a material that is transparent and has an appropriate flexibility, for example, polypropylene, so as to be thin in a range capable of maintaining the strength. In addition, the inside diameter of the pressing ring 60 is formed to be substantially equal to the outside diameter of the outer peripheral surface of the safety device body 51, and accordingly, the pressing ring 60 may slide on the outer peripheral surface of the safety device body 51 along the direction of the axis O.

In addition, in this embodiment, only when the pressing ring 60 is moved to a position corresponding to the return prevention stoppers 54 on the outer peripheral surface of the safety device body 51, the return prevention stopper 54 protrudes inward in the radial direction. In addition, when the pressing ring 60 is moved to a position other than the position corresponding to the return prevention stopper 54 on the outer peripheral surface of the safety device body 51, the return prevention stopper 54 returns toward the outside in the radial direction, and the state of the return prevention stopper 54 protruding inward in the radial direction is released.

In addition, the rear end side of the slip-out prevention stopper 53 in the inner peripheral surface of the safety device body 51 has a diameter increased compared to those of other positions, and a step portion formed by the increase in the diameter so as to be directed rearward is a spring abutting step portion 51a on which the tip end of the coil spring 59 abuts.

In the safety device body 51, a pair of engagement plates (engagement portions) 58 which have rectangular plate shapes extending in parallel to the axis O from the rear end of the outer peripheral surface to the rear side are formed at an interval of 180° in the peripheral direction so as to oppose each other in parallel. The engagement plates 58 are elastically deformable along the radial direction, and specifically, as illustrated in FIG. 2, have circular engagement holes 58b which are formed to penetrate in the thickness direction (radial direction). The inside diameter of the engagement hole 58b is formed to be substantially equal to the outside diameter of the fastening convex portion 44 formed in the fitting portion 41 of the finger grip 40 in the main body of combined container-syringe 10, and as the fastening convex portion 44 is fitted into the engagement hole 58b, the fastening convex portion 44 fastens the engagement plate 58.

That is, in this embodiment, the first holding means 3 is composed of the fastening convex portions 44 and the engagement plates 58. In addition, in order to release the fastening of the engagement plate 58 and the fastening convex portion 44, the engagement plate 58 may be elastically deformed outward to disengage the fastening convex portion 44 from the engagement hole 58b.
In addition, in a state where the safety device 50 is moved forward and fully covers the injection needle 37, the slip-out prevention stopper 53 and the return prevention stopper 54 are engaged with the safety device engagement portion 31 of the hub luer-lock 30. Accordingly, the safety device 50 is held on the front side of the main body of combined container-syringe 10. That is, in this embodiment, the second holding means 4 is composed of the slip-out prevention stopper 53, the return prevention stopper 54, and the safety device engagement portion 31.

In addition, specifically, as illustrated in FIG. 2, on the surface directed outward in the radial direction in each of the engagement plates 58 of the safety device body 51, two ribs 58c extending in parallel to the axis O are formed.

The safety device 50 having the configuration as described above is, as illustrated in FIG. 1, fitted to the main body of combined container-syringe 10 from the outside. In addition, in the safety device 50 in this state, in the state where the return prevention stopper 54 is pressed by the pressing ring 60, the return prevention stopper 54 accordingly protrudes inward in the radial direction.

In addition, the engagement holes 58b and 58b of the pair of the engagement plates 58, 58 of the safety device 50 are respectively fitted into the fastening convex portions 44 and 44 of the finger grip 40, so that the engagement plates 58, 58 and the fastening convex portions 44 and 44 are fastened to each other. That is, the safety device 50 is caused to be in the fastened state by the first holding means 3.

In addition, the coil spring 59 is accommodated in a compressed state by causing the rear end thereof to abut on the spring abutting step portion 41 a in the finger grip 40 and causing the front end thereof to abut on the spring abutting step portion 51 in the safety device 50. Here, although a biasing force is generated by the coil spring 59, since the engagement plate 58 and the fastening convex portion 44 are in the fastened state as described above, the movement of the safety device 50 is impeded.

In addition, as such, in the state of the safety device 50 is attached to the outer periphery of the main body of combined container-syringe 10, the rear end portion of the safety device body 51 is accommodated in the inside in the radial direction of the pair of the finger contact covers 49 and 49.

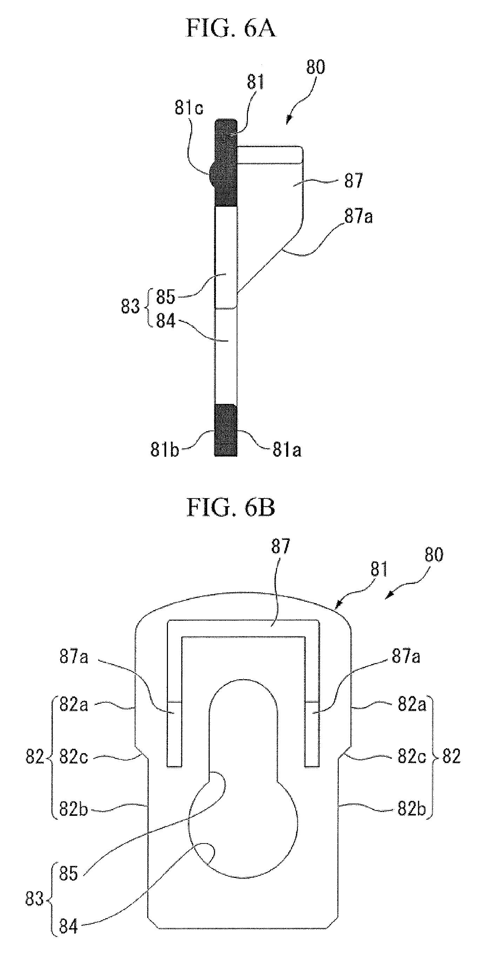

Next, the fastening releasing means 80 for releasing the fastening by the first holding means 3 will be described. The fastening releasing means 80 is disposed to be slidable on the flange surface 46 of the finger grip 40. In addition, as illustrated in FIGS. 6A and 6B, the fastening releasing means 80 includes a slide plate 81 having a substantially rectangular plate shape, and an operation plate 87 which extends vertically from a front plate surface (a surface directed to the rear side of the combined container-syringe 1) 81 a of the slide plate 81.

The slide plate 81 has a substantially rectangular shape aligned with the longitudinal direction and the width direction of the flange portion 45. In addition, a surface on the opposite side to the front plate surface 81 a in the slide plate 81 is a rear plate surface 81b, and as the rear plate surface 81b comes into contact with the flange surface 46, the rear plate surface 81b may slide on the flange surface 46 in the longitudinal direction. In addition, at a position close to one side in the longitudinal direction of the rear plate surface 81 b, a slide plate fastening portion 81 c protruding in a convexly curved surface shape. The slide plate fastening portion 81 c may be engaged with the fastening concave portion 46a of the flange surface 46 of the finger grip 40.

In addition, the interval between the pair of side surfaces 82 and 82 positioned at both end portions of the width direction of the slide plate 81 is reduced toward the other side from the one side in the longitudinal direction. That is, the side surfaces 82 and 82 include first side surfaces 82a and 82a that are disposed close to the one side and are separated from each other at a predetermined interval, and second side surfaces 82b and 82b that are positioned close to the other side and are separated from each other at a smaller interval than that of the first side surfaces 82a and 82a. In addition, boundary portions between the first side surfaces 82a and 82a and the second side surfaces 82b and 82b are inclined side surfaces 82c and 82c that are gradually inclined in a direction to approach each other from the one side to the other side in the longitudinal direction, that is, in the radial direction in a state where the flange surface 46 is disposed.

In addition, as illustrated in FIGS. 6A and 6B, in the slide plate 81, a through-hole 83 is formed which penetrates through the rear plate surface 81b from the front plate surface 81 a. The through-hole 83 is composed of a large diameter hole 84 having a predetermined inside diameter, and a small diameter slit 85 that extends continuously from the large diameter hole 84 toward the one side in the longitudinal direction while maintaining the inside diameter smaller than that of the large diameter hole 84.

In addition, further towards the one side than the through-hole 83 of the slide plate 81 as described above, the operation plate 87 having a substantially U shape as viewed from the rear side so as to surround the small diameter slit 85 of the through-hole 83 is provided.
The operation plate 87 is composed of a pair of vertical plates extending along the longitudinal direction at both end portions of the small diameter slit 85 and a horizontal plate that connects the vertical plates to each other in the width direction at an end portion on the one side in the longitudinal direction of the vertical plates. In addition, at end portions on the other side in the longitudinal direction of the vertical plates, inclined surfaces 87a are respectively formed which are gradually inclined rearward from the other side to the one side in the longitudinal direction.

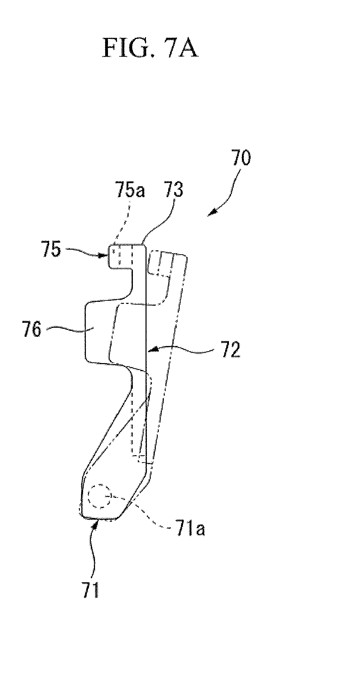

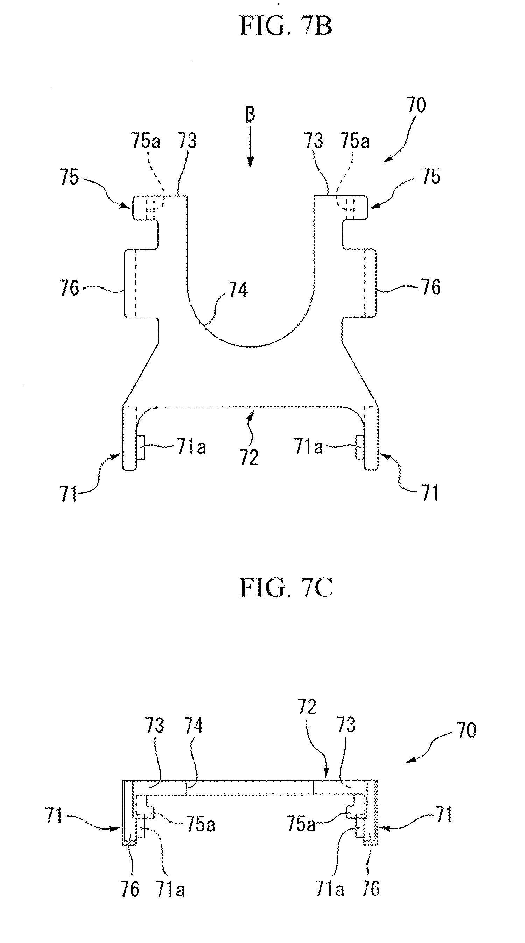

Next, the locking means 70 for locking the fastened state of the safety device 50 will be described with reference to FIGS. 7A to 7C. In addition, the upward and downward direction in FIG. 7B is the longitudinal direction of the locking means 70, and the left and right direction is the width direction of the locking means 70.

The locking means 70 is disposed so that the longitudinal direction and the width direction thereof are aligned with those of the flange portion 45 and the fastening releasing means 80. At both end portions on the other side in the longitudinal direction in the locking means 70, attaching portions 71 are respectively formed which are disposed to oppose each other in the width direction. On the surfaces of the attaching portions 71 opposing each other, columnar protrusions 71a are respectively formed. As the columnar protrusions 71 a are fitted to the attaching holes 48c in the flange portion 45, the locking means 70 is attached to the flange portion 45 so as to be rotatable around an axis P along the direction thereof.

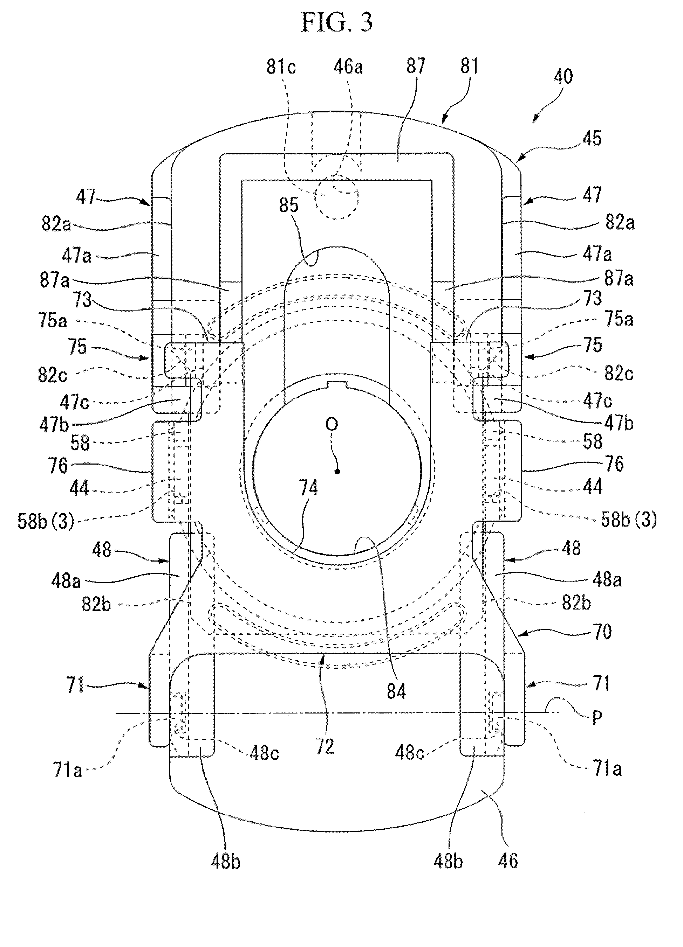

In addition, a locking means main body 72 having a plate shape is formed integrally with the pair of the attaching portions 71. The locking means main body 72 is disposed to overlap with the flange portion 45 and the fastening releasing means 80, and an end portion on the one side in the longitudinal direction thereof is an abutting surface 73 on which the inclined surfaces 87a and 87a of the fastening releasing means abut when the fastening releasing means 80 slides. In addition, the locking means main body 72 is provided with a slit hole 74 formed from the abutting surface 73 toward the other side. The slit hole 74 has substantially the same diameter as that of the through-hole 43 in the finger grip 40, and as illustrated in FIG. 3, the through-hole 43 and the slit hole 74 are disposed so that the circumferences thereof are aligned with each other.

In addition, at both end portions on the one side in the longitudinal direction of the locking means main body 72, that is, both end portions of the abutting surface 73, a pair of fixing portions 75 and 75 is provided for fixing the position of the locking means main body 72 in the state where the locking means main body 72 overlaps with the flange portion 45 and the fastening releasing means 80. On the surfaces of the fixing portions 75 and 75 opposing each other, nail-shaped protrusions 75a and 75a are respectively provided, and as the nail-shaped protrusions 75a and 75a are fitted into the rectangular grooves 47c in the flange portion 45, the locking means 70 is fixed.

In addition, on both sides in the longitudinal direction of the locking means main body 72, a plurality of pressing portions 76 and 76 extending forward in the state where the locking means main body 72 overlaps with the flange portion 45 and the fastening releasing means 80 are formed integrally.
The interval between the pressing portions 76 and 76 is smaller than the interval between the ribs 58c of the pair of the engagement plates 58, 58 in the safety device body 51. Accordingly, when the pressing portions 76 and 76 abut from the outside in the radial direction of the engagement plates 58, 58, the pressing portions 76 and 76 respectively press the ribs 58c of the engagement plates 58, 58 from the outside in the radial direction.

Next, a state where the fastening releasing means 80 and the locking means 70 are attached to the finger grip 40 will be described in detail. As illustrated in FIGS. 2 to 4, the fastening releasing means 80 and the locking means 70 are disposed to overlap on the flange portion 45 of the finger grip 40 in the order of the fastening releasing means 80 and the locking means 70.

As illustrated in FIGS. 2 and 3, the fastening releasing means 80 is disposed close to the one side in the longitudinal direction of the flange surface 46 by causing the rear plate surface 81b to come into surface contact with the flange surface 46 of the flange portion 45. Here, the pair of the first side surfaces 82a and 82a of the fastening releasing means 80 respectively come into contact with the side guides 47a and 47a of the first guide portions 47 and 47 of the flange portion 45, and the pair of the second side surfaces 82a and 82a of the fastening releasing means 80 come into contact with the side guides 48a and 48a of the second guide portions 48 and 48. In addition, the second side surfaces 82b and 82b of the fastening releasing means 80 respectively come into contact with the inner side surfaces of the engagement plates 58, 58 of the safety device 50. Moreover, in the through-hole 83 of the fastening releasing means 80, the center of the large diameter hole 84 is aligned with the axis O, and accordingly, the large diameter hole 84 and the through-hole 43 of the rear end of the finger grip 40 are in a communicating state.

As such, the state where the second side surfaces 82b and 82b come into contact with the engagement plates 58, 58 and the center of the large diameter hole 84 is aligned with the axis O is a standby position at which the fastening releasing means 80 is positioned close to the outside in the radial direction of the combined container-syringe 1. At this standby position, the fastening releasing means 80 is fastened onto the flange surface 46 as the slide plate fastening portion 81 c is fitted into the fastening concave portion 46a of the flange surface 46.

In addition, as illustrated in FIGS. 2 and 3, the locking means 70 is disposed so that the columnar protrusions 71a of the attaching portions 71 and 71 are respectively fitted into the attaching holes 48c of the flange portion 45, and the nail-shaped protrusions 75a and 75a of the fixing portions 75 and 75 are engaged with the rectangular grooves 47c and 47c of the flange portion 45. In addition, the locking means 70 is rotatable around the columnar protrusions 71 a and 71 a, that is, around the axis P, and in this state, the locking means 70 is not rotated by the fixing portions 75 and 75. In addition, the abutting surface 73 of the locking means 70 disposed as such abuts on the inclined surface 87a of the fastening releasing means 80.

Moreover, in this state, the pair of the pressing portions 76 and 76 in the locking means 70 respectively press the ribs 58c of the pair of the engagement plates 58, 58 in the safety device 50 from the outside in the radial direction. Since the identical fastening convex portions 44 and 44 of the finger grip 40 are fitted into the engagement holes 58b of the engagement plates 58, 58, engagement between the engagement plates 58, 58 and the fastening convex portions 44 and 44, that is, the fastened state of the safety device 50 by the first holding means 3 at the retreated position is restricted by the pressing portions 76 and 76 of the locking means 70.

Next, an operation when the safety device 50 is moved to the advanced position from the retreated position will be described.
After completing injection, in order to cover the injection needle 37 with the safety device 50, the fastening releasing means 80 is slid to the release position positioned on the other side in the longitudinal direction from the standby position along the longitudinal direction. This operation is performed by operating the operation plate 87 of the fastening releasing means 80 with, for example, a finger tip. Here, the fastening releasing means 80 is guided by the first guide portions 47 and 47 and the second guide portions 48 and 48 provided in the flange portion 45 and is thus smoothly slid.

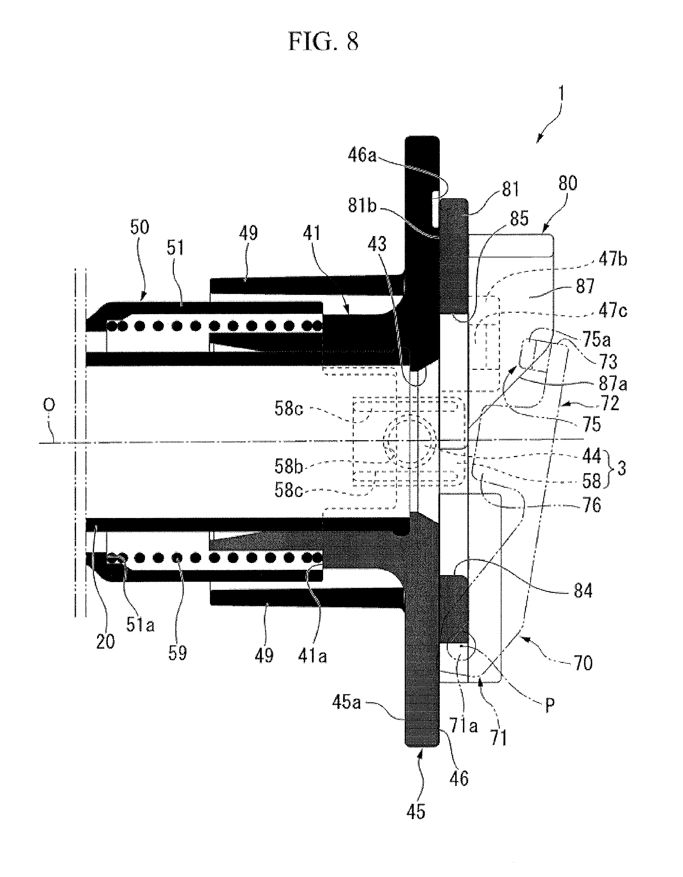

During the sliding movement, first, the inclined surface 87a of the fastening releasing means 80 abut on the abutting surface 73 in the locking means 70. When the fastening releasing means 80 is further slid toward the release position from the state where the inclined surface 87a and the abutting surface 73 abut on each other, as illustrated in FIG. 8, the nail-shaped protrusion 75a of the locking means 70 is deviated from the rectangular groove 47c of the finger grip 40, the abutting surface 73 rides along the inclined surface 87a, and accordingly the locking means 70 is turned to the rear side around the columnar protrusion 71 a of the attaching portion 71, that is, around the axis P. Accordingly, the pressing portion 76 of the locking means 70 is deviated from the state of pressing the engagement plate 58 of the safety device 50, so that restriction of the engagement plate 58 by the locking means 70 is released.

In addition, when the fastening releasing means 80 is further slid toward the release position, the inclined side surfaces 82c and 82c of the fastening releasing means 80 abut on the engagement plates 58, 58 of the safety device 50, and the engagement plates 58, 58 are bent outward in the radial direction so as to ride on the inclined side surfaces 82c and 82c. Then, the engagement holes 58b of the engagement plates 58, 58 are deviated from the fastening convex portions 44 of the finger grip 40, that is, restriction of the safety device 50 by the first holding means 3 at the retreated position is released.

As a result, the safety device 50 is moved forward by the biasing force of the coil spring 59. In addition, at a position where the safety device 50 moved as such covers the entire area of the injection needle 37, the slip-out prevention stopper 53 and the return prevention stopper 54 are engaged with the safety device engagement portion 31. Accordingly, holding and fixing the safety device 50 at the advanced position by the second holding means 4 is completed, so that the combined container-syringe 1 may be safely treated without exposure of the injection needle 37 to the outside.

As such, according to the combined container-syringe 1 of this embodiment, by moving the fastening releasing means 80 in the radial direction, the fastened state of the safety device by the first holding means 3 may be released. Accordingly, the safety device 50 is moved forward by the biasing of the coil spring 59 and covers the injection needle 37. That is, since the safety device 50 may be moved forward only by the operation of moving the fastening releasing means 80 to slide, the injection needle 37 may be covered by the safety device 50 easily and reliably. In addition, since the fastened state of the safety device 50 by the first holding means 3 is locked by the locking means 70, for example, even in a case where an external force is abruptly exerted to release the fastened state of the safety device 50 by the first holding means 3, this fastened state is not released. Therefore, safety may be further ensured.

In addition, by a single sliding movement of the fastening releasing means 80, the locking of the fastened state of the safety device 50 by the locking means 70 and the fastened state of the safety device 50 by the fastening releasing means 80 may be sequentially released. Therefore, the safety device 50 may be operated with an easy operation while reliably preventing a malfunction of the safety device 50.

Moreover, as the engagement plates 58, 58 on the safety device 50 side are engaged with the locking convex portions 44 of the finger grip 40 from the outside in the radial direction, the safety device 50 may be reliably in the fastened state at the retreated position. In addition, as the pressing portions 76 and 76 of the locking means 70 further press the engagement plates 58, 58 from the outside in the radial direction, the fastened state may be reliably locked.

In addition, since the pressing portions 76 and 76 of the locking means 70 press the ribs 58c of the engagement plates 58, 58, contact pressure between the pressing portions 76 and 76 and the engagement plats 58, 58 is increased. Therefore, the locking of the fastened state by the pressing portions 76 and 76 may be reliably performed.

Here, in a case where the finger of a doctor, a nurse, or the like comes into contact with the outer peripheral portion of the rear end of the safety device 50 when the locking of the fastened state by the locking means 70 and the fastened state by the first holding means 3 is released, there is a possibility that forward movement of the safety device 50 by the biasing of the coil spring 59 may be impeded, and the safety device 50 may not be normally operated.
From this point, according to this embodiment, since the finger contact covers 49 and 49 which surround and cover the outer peripheral portion of the rear end of the safety device 50 from the outer peripheral side are provided, the finger of the doctor, nurse, or the like comes into contact with the finger contact covers 49 and 49 and does not come into contact with the safety device 50. Accordingly, the operation of the safety device 50 is not impeded, and the injection needle 37 may be reliably covered by the safety device 50.

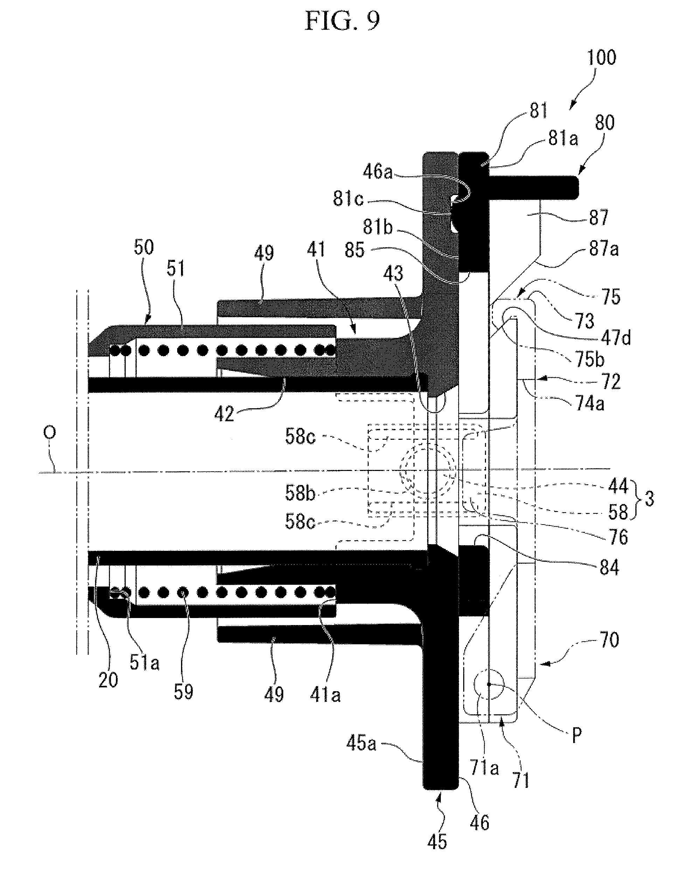

Next, a combined container-syringe 100 of a second embodiment of the invention will be described with reference to FIGS. 9 to 15. In addition, in FIGS. 9 to 15, like elements which are the same as those of the combined container-syringe 1 of the first embodiment are denoted by like reference numerals, and detailed description thereof will be omitted.
In the combined container-syringe 100 of the second embodiment, a fixing method of the locking means 70 and the finger grip 40 is different from that of the first embodiment.

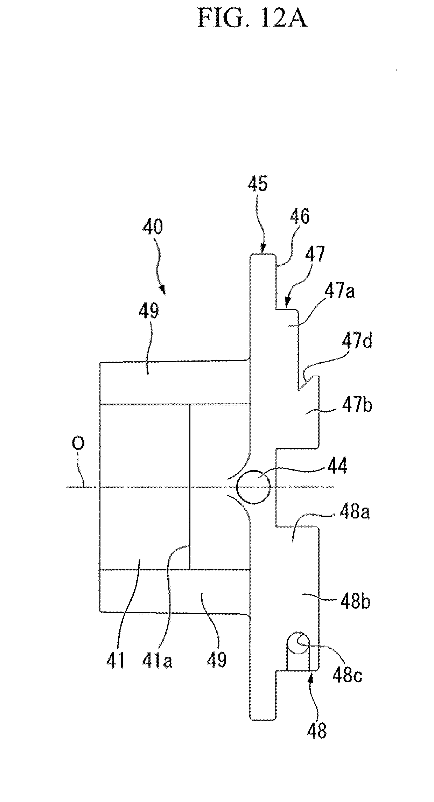

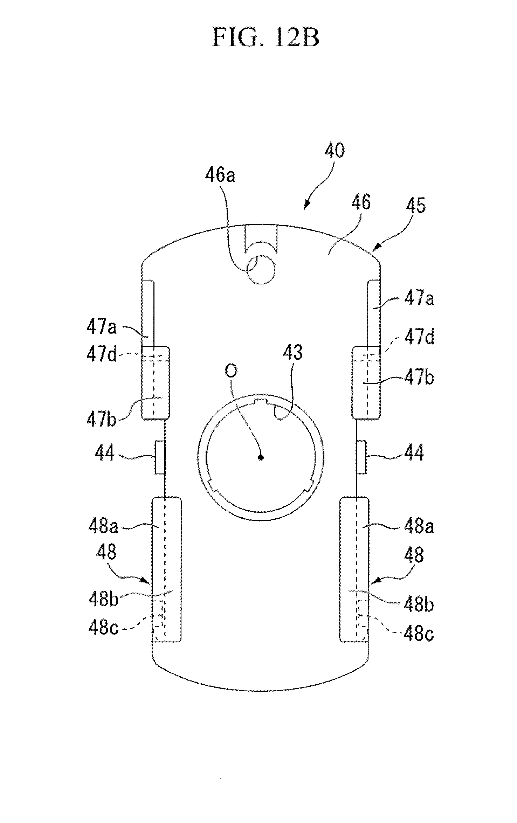

As illustrated in FIGS. 12A and 12B, in the finger grip 40 of the second embodiment, at positions directed to the one side in the longitudinal direction of the flange portion 45 in the pressing guides 47b of the first guide portions 47 and 47, acute angle grooves 47d and 47d formed at acute angles are formed.

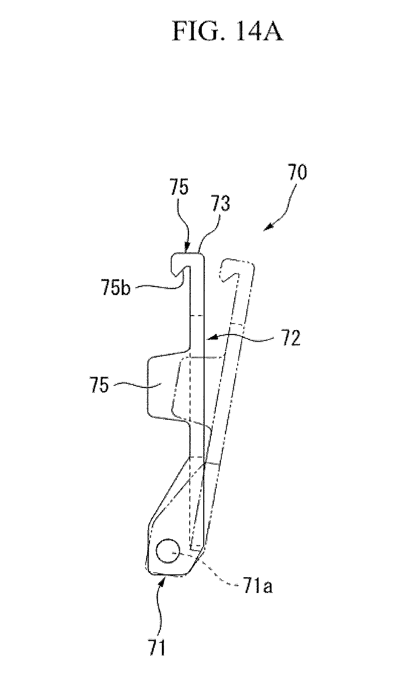

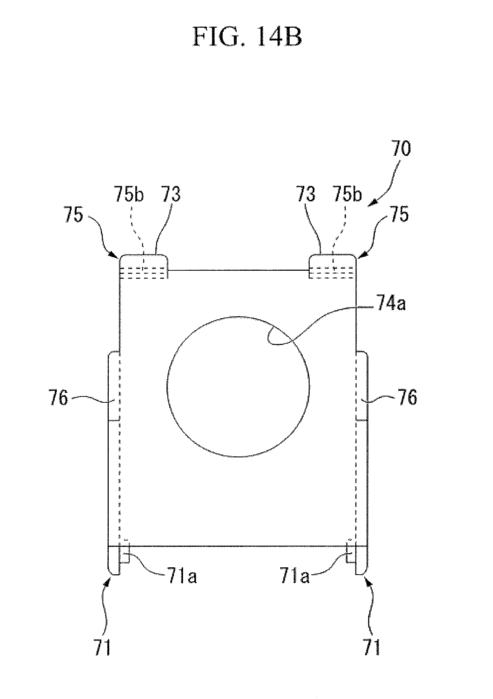

In addition, the locking means 70 of the second embodiment has a rectangular flat plate shape as illustrated in FIGS. 14A and 14B, and in the locking means 70, instead of the slit hole 74 in the first embodiment, a circular through-hole 74a is formed. In addition, at both end portions of the width direction of the one side in the longitudinal direction in the locking means main body 72, engagement nails 75b and 75b which are engaged with the acute angle grooves 47d and 47d formed in the finger grip 40 from the one side in the longitudinal direction are formed. In addition, a part directed to the one side in the longitudinal direction in the engagement nails 75b and 75b are the abutting surface 73 on which the inclined surfaces 87a and 87a of the fastening releasing means 80 abut.

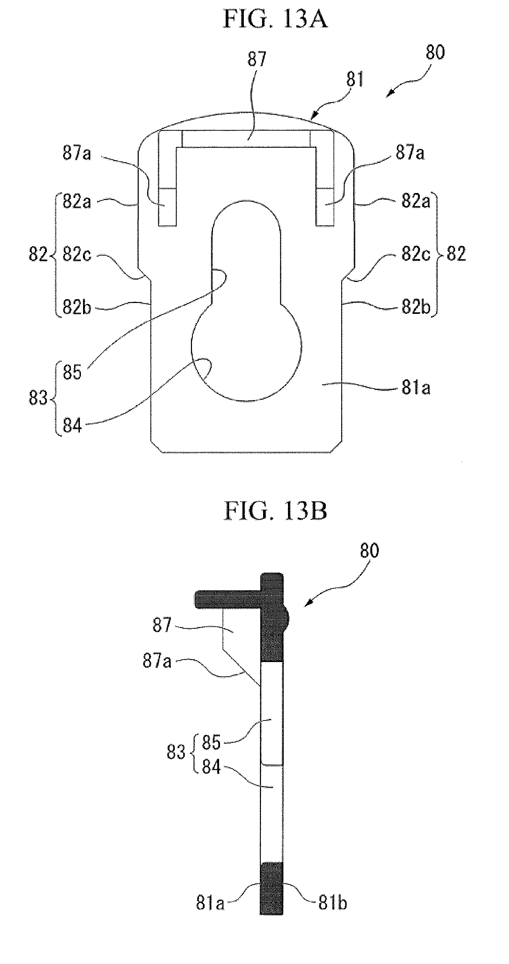

On the other hand, as illustrated in FIGS. 13A and 13B, the fastening releasing means 80 has substantially the same shape as that of the first embodiment. That is, the fastening releasing means 80 includes the slide plate 81 having a substantially rectangular plate shape, and the operation plate 87 extending vertically from the front plate surface 81a of the slide plate 81. In addition, at the boundary portions between the first side surfaces 82a and 82a and the second side surfaces 82b and 82b, the inclined side surfaces 82c and 82c are formed. Moreover, in the slide plate 81, the through-hole 83 composed of the large diameter hole 84 and the small diameter slit 85 is formed.

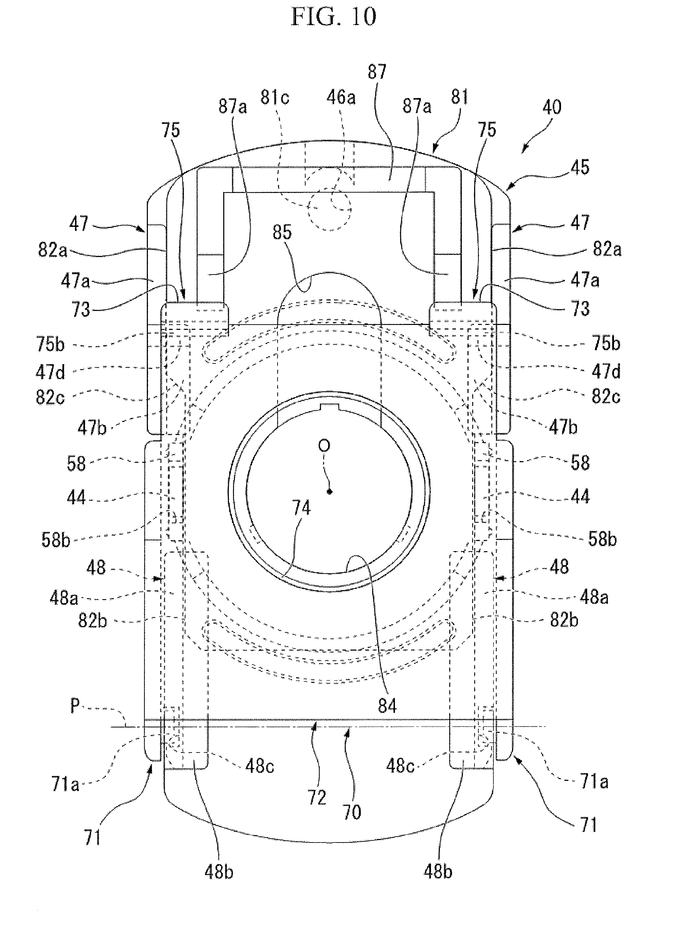

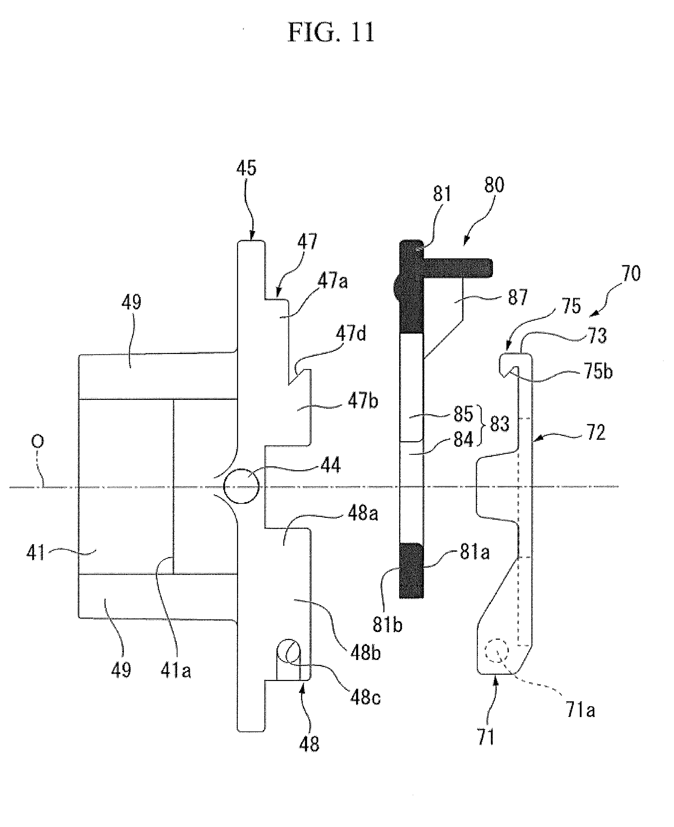

Even in the second embodiment, like the first embodiment, as illustrated in FIG. 11, on the flange surface 46 of the flange portion 45 of the finger grip 40, the fastening releasing means 80 and the locking means 70 are disposed to overlap in this order. In addition, in the disposition state as such, as illustrated in FIGS. 9 and 10, the pair of the engagement nails 75b and 75b in the locking means 70 are engaged with the acute angle grooves 47d and 47d of the finger grip 40, thereby fixing the locking means 70.

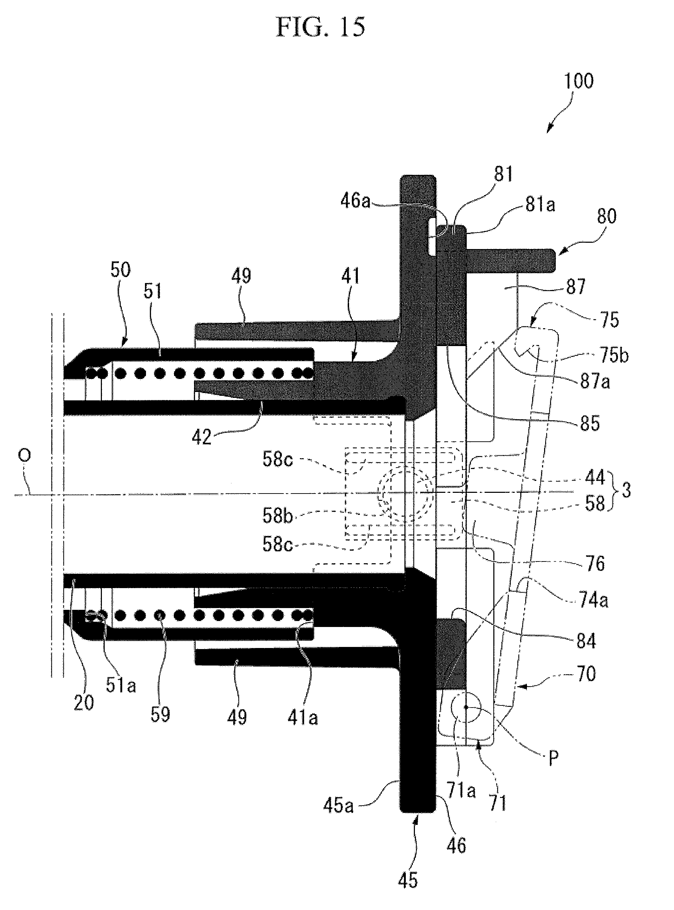

In the combined container-syringe 100 of the second embodiment, when the fastening releasing means 80 is slid from the standby position to the release position, first, the inclined surfaces 87a of the fastening releasing means 80 abuts on the abutting surface 73 in the locking means 70. From the state where the inclined surface 87a and the abutting surface 73 abut on each other as such, when the fastening releasing means 80 is slid toward the release position, as illustrated in FIG. 15, the engagement of the engagement nails 75b and 75b of the locking means 70 and the acute angle grooves 47d and 47d of the finger grip 40 is released, and the abutting surface 73 of the locking means 70 rides on the inclined surface 87a, such that the locking means 70 is accordingly turned to the rear side about the axis P. Accordingly, the pressing portion 76 of the locking means 70 is deviated from the state of pressing the engagement plate 58 of the safety device 50, and the restriction of the engagement plate 58 by the locking means 70 is released.

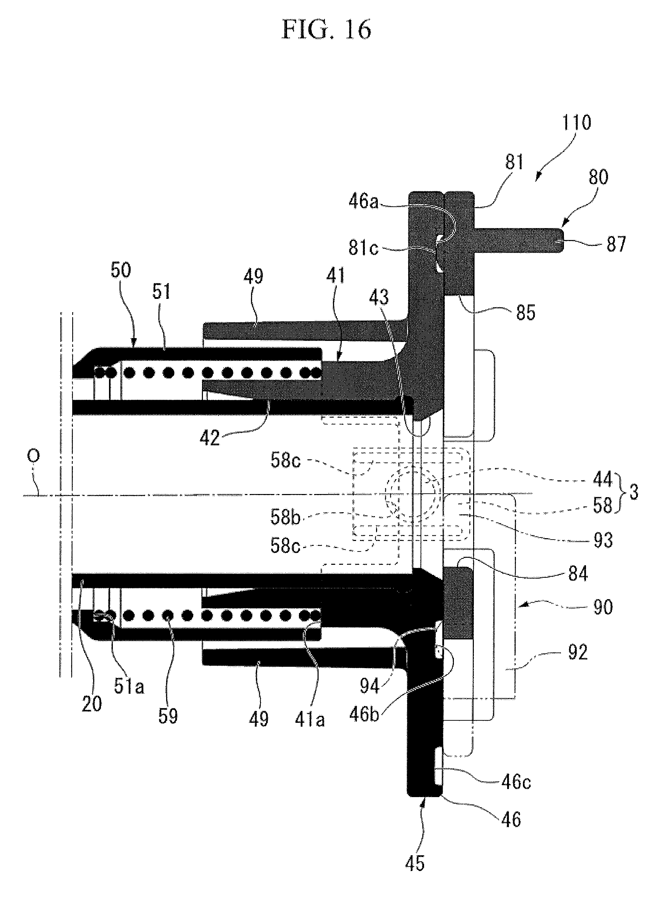

Next, a combined container-syringe 110 of the third embodiment will be described with reference to FIGS. 16 to 21. In addition, in FIGS. 16 to 21, like elements which are the same as those of the combined container-syringe 1 of the first embodiment are denoted by like reference numerals, and detailed description thereof will be omitted.
In the combined container-syringes 1 and 100 of the first and second embodiments, the locking means 70 is attached to the finger grip 40 so as to be rotatable around the axis P, whereas in the combined container-syringe 110 of the third embodiment, the locking means 70 is disposed to be slidable on the flange surface 46 of the finger grip 40.

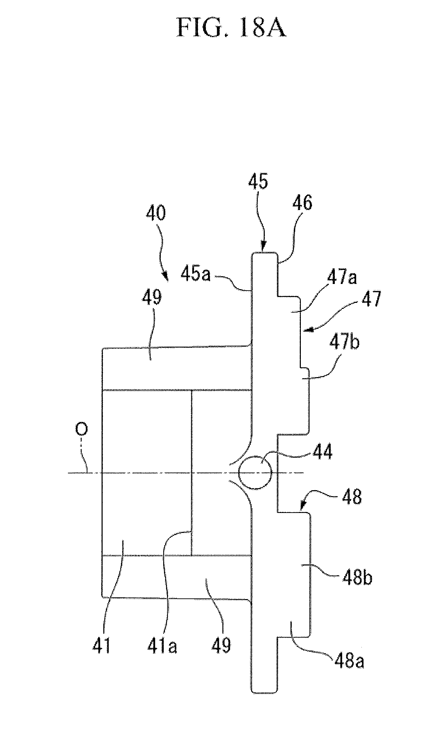

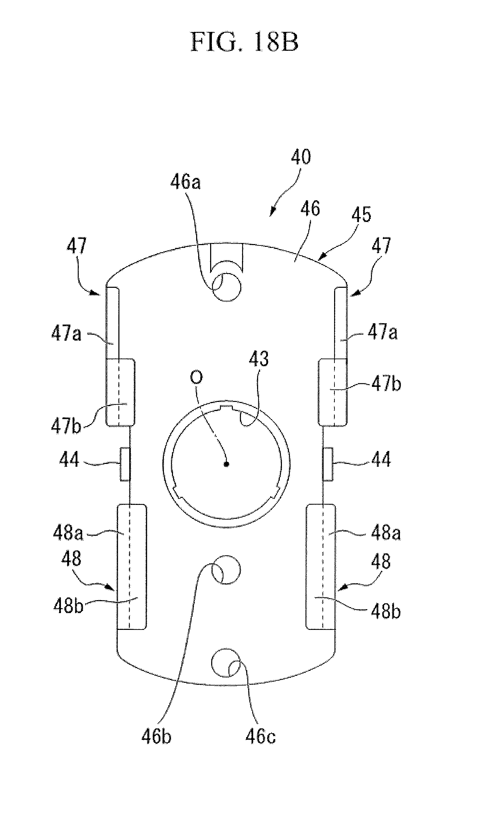

As illustrated in FIGS. 18A and 18B, in the flange surface 46 of the finger grip 40 of the third embodiment, a second fastening concave portion 46b and a third fastening concave portion 46c having concave hole shapes are formed on the other side in the longitudinal direction.
The second fastening concave portion 46b is positioned at a position close to the insertion hole 43, and the third fastening concave portion 46c is disposed at a position distant from the insertion hole 43 further towards the other side in the longitudinal direction than the second fastening concave portion 46b.

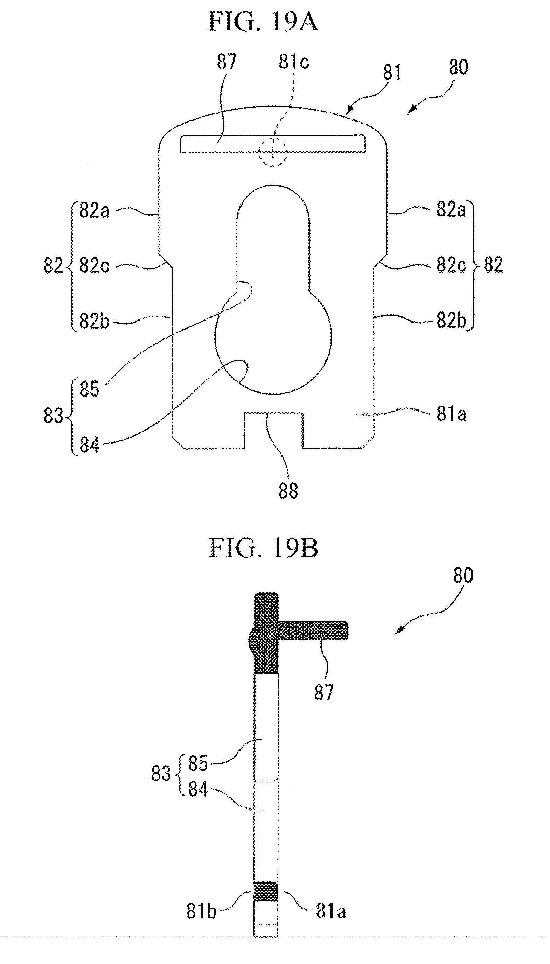

As illustrated in FIGS. 19A and 19B, in the operation plate 87 in the fastening releasing means 80 in the third embodiment, the inclined surface 87a as in the first and second embodiments is not formed, and the operation plate 87 has a plate shape disposed along the width direction. In addition, in the fastening releasing means 80, at a center portion of the width direction of the surface on the other side in the longitudinal direction, a rectangular slit 88 having a rectangular shape in a plan view is formed.

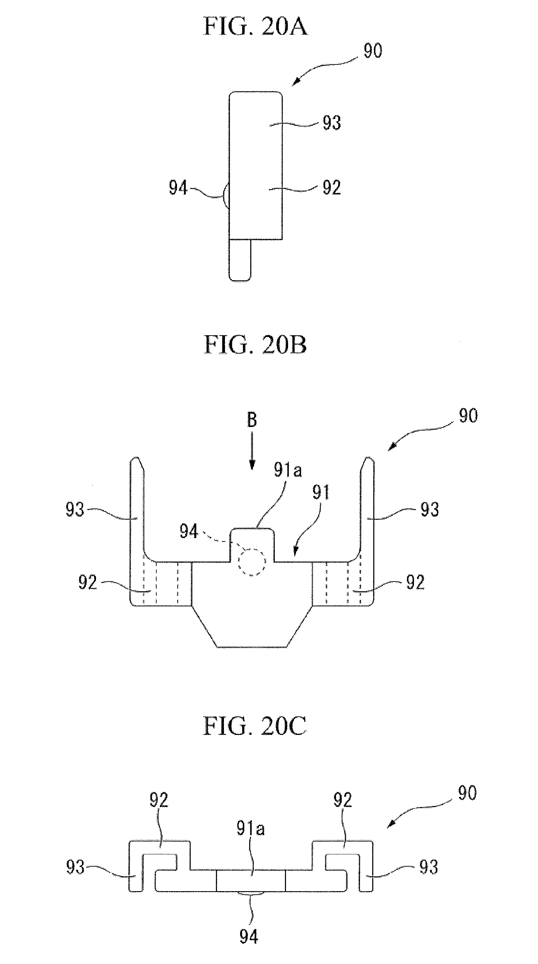

The structure of the locking means 90 in the third embodiment is shown in FIGS. 20A to 20C. The locking means 90 has a substantially U shape in a plan view as illustrated in FIG. 20B. In addition, the locking means 90 includes a fastening releasing means engagement portion 91, guide engagement portions 92 and 92 provided on both sides of the fastening releasing means engagement portion 91, and pressing portions 93 and 93 respectively extending from the guide engagement portions 92 and 92.

The fastening releasing means engagement portion 91 is a part engaged with the surface of the other side in the longitudinal direction of the fastening releasing means 80, and at the center portion of the fastening releasing means engagement portion 91, an engagement rectangular portion 91a fitted into the rectangular slit 88 in the fastening releasing means 80 is formed.

In addition, the guide engagement portions 92 and 92 are parts engaged with the second guide portions 48 in the finger grip 40 to be slidable along the slide direction and have substantially L shapes when viewed in cross-section so as to be fitted to the second guide portions 48.

Moreover, the pressing portions 93 and 93 are members that press the engagement plate 58 of the safety device 50 from the outside in the radial direction, and are configured to press the engagement plate 58 when the locking means 90 is positioned at the initial position and to release the pressed state of the engagement plate 58 when the locking means 90 is moved to a movement position deviated from the initial position.
In addition, on the rear surface side of the locking means 90, an engagement protrusion 94 which is engaged with the second fastening concave portion 46b or the third fastening concave portion 46c in the flange surface 46 is provided.

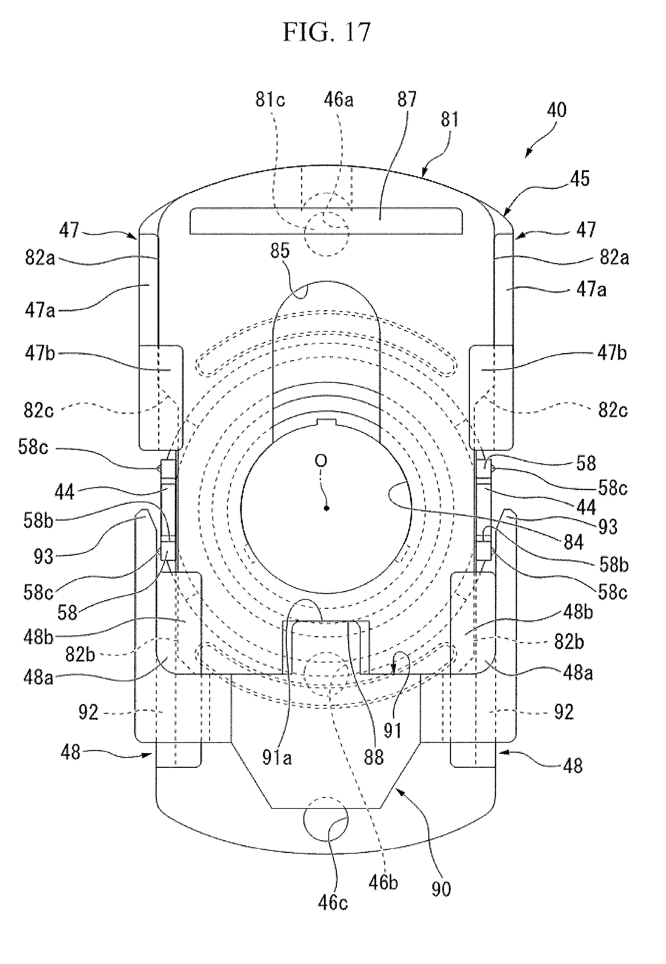

In the third embodiment, as illustrated in FIGS. 16 and 17, the fastening releasing means 80 is disposed on the one side in the longitudinal direction of the flange surface 46 of the finger grip 40, and the locking means 90 is disposed on the other side in the longitudinal direction. Here, in the locking means 90, the guide engagement portions 92 and 92 are fitted to the second guide portions 48 and 48, and accordingly, the locking means 90 may slide along the longitudinal direction of the flange surface 46. In addition, the fastening releasing means 80 abuts on the fastening releasing means engagement portion 91 in the locking means 90, and here, the engagement rectangular portion 91 a of the locking means 90 is fitted into the rectangular slit 88 of the fastening releasing means 80.

In addition, in the locking means 90 abutting on the fastening releasing means 80 as described above, the engagement protrusion 94 of the locking means 90 is engaged with the second fastening concave portion 46b of the flange surface 46 in the state where the fastening releasing means 80 is at the standby position. Accordingly, the locking means 90 is fixed at the initial position and is not moved by a small external force.

Even in the combined container-syringe 110 of the third embodiment, in order to operate the safety device 50, the fastening releasing means 80 is slid from the standby position to the release position located on the other side in the longitudinal direction along the radial direction. Here, the fastening releasing means 80 is guided by the first guide portions 47 and 47 and the second guide portions 48 and 48 provided in the flange portion 45 and is thus smoothly slid.

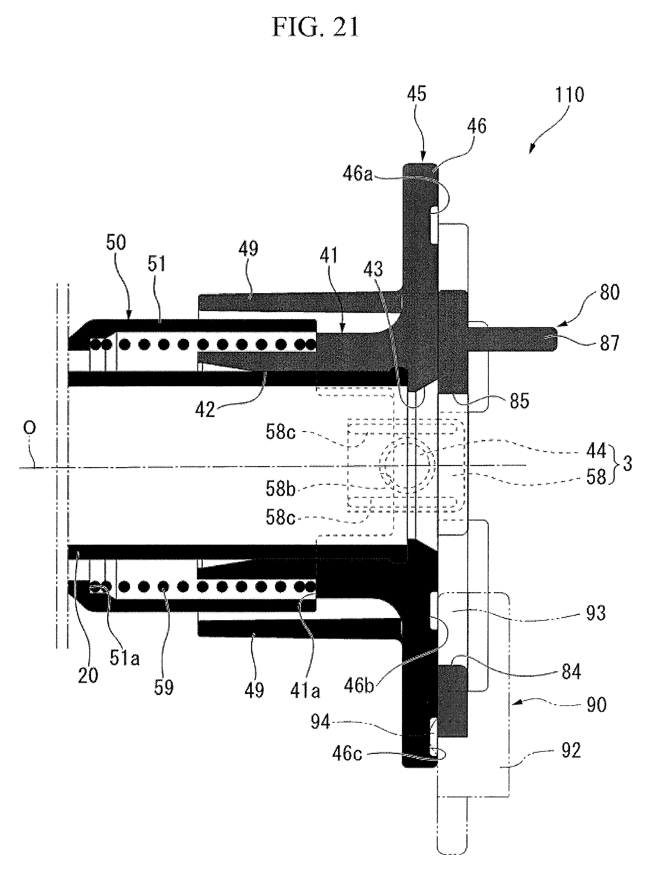

In addition, during the sliding movement, the locking means 90 abutting on the fastening releasing means 80 is also slid toward the other side in the longitudinal direction of the flange surface 46, like the fastening releasing means 80. Then, as illustrated in FIG. 21, the pressing portions 93 and 93 of the locking means 90 are deviated from the engagement plates 58, 58 of the safety device 50, and thus the pressed state of the engagement portions 58, 58 by the pressing portions 93 and 93 is released.

In addition, when the fastening releasing means 80 is further slid toward the release position, the inclined side surfaces 82c and 82c of the fastening releasing means 80 abut on the engagement plates 58, 58 of the safety device 50, and the engagement plates 58, 58 are bent outward in the radial direction so as to ride on the inclined side surfaces 82c and 82c. Then, the engagement holes 58b and 58b of the engagement plates 58, 58 are deviated from the fastening convex portions 44 of the finger grip 40, that is, restriction of the safety device 50 by the first holding means 3 at the retreated position is released, and the safety device 50 is moved forward.

As such, even in the combined container-syringe 110 of the third embodiment, as the fastening releasing means 80 is slid, first, the locking by the first locking means 90 is released, and second, the fastened state of the safety device 50 by the first holding means 3 may be released. Therefore, when the combined container-syringe 110 is used, safety and convenience may be ensured.

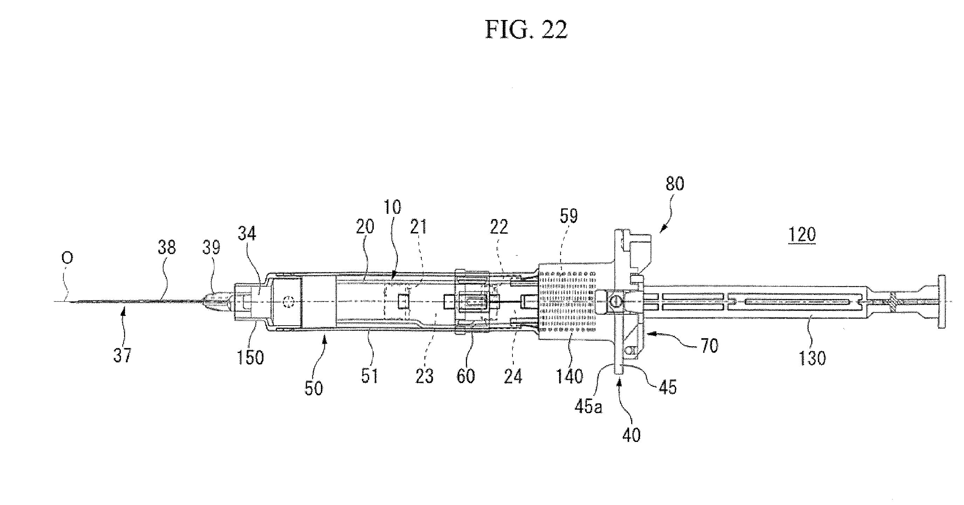

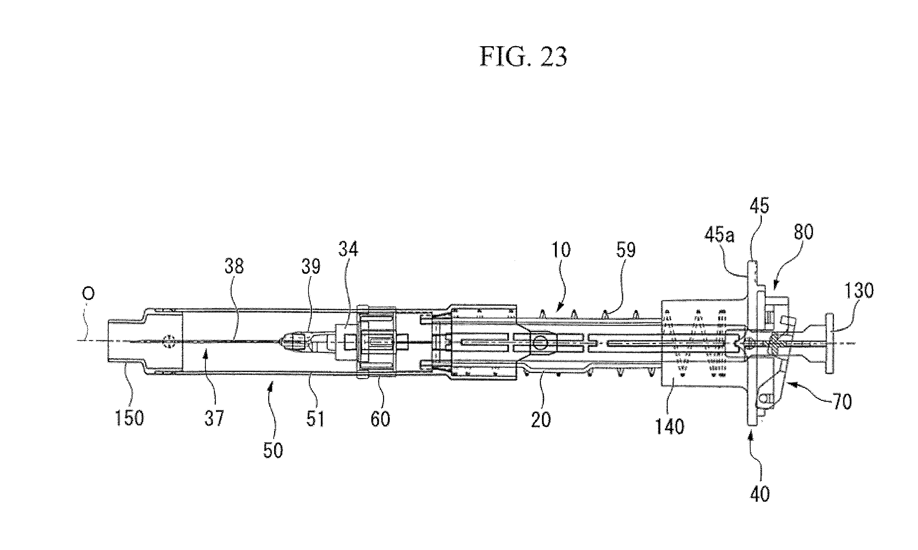

Next, a combined container-syringe 120 of the fourth embodiment of the invention will be described with reference to FIGS. 22 and 23. In addition, in FIGS. 22 and 23, like elements which are the same as those of the combined container-syringe 1 of the first embodiment are denoted by like reference numerals, and detailed description thereof will be omitted.

In the combined container-syringe 120, inside the outer cylinder 20, a front stopper 21, a middle stopper 22, and a rear stopper (not illustrated) are liquid-tightly provided from front at intervals along the direction of the axis O. In addition, to the rear end of the rear stopper, the front end of a plunger rod 130 is fixed. The plunger rod 130 penetrates through the finger grip 40, the locking means 70, and the fastening releasing means 80 and extends rearward along the axis O. In addition, in spaces 23 and 24 respectively formed between the front stopper and the middle stopper and between the middle stopper and the rear stopper, medicine and medical solution are respectively sealed.

In addition, in the combined container-syringe 120 of the fourth embodiment, a finger contact cover 140 of the finger grip 40 extends further toward the front side from the finger hooking surface 45a of the flange portion 45 than the finger contact cover 49 of the first to third embodiments and covers the fitting portion 41 and the outer peripheral surface of the rear end portion (a part where the coil spring 59 is accommodated) of the safety device body 51 with a gap from the outer peripheral surfaces thereof. In addition, unlike the finger contact cover 49, the finger contact cover 140 has a cylindrical shape surrounding the periphery of the fitting portion 41 and the safety device body 51.

In the combined container-syringe 120, the fitting portion 41 and the rear end portion of the safety device body 51 are completely covered by the finger contact cover 140 having the cylindrical shape from the outside. As a result, compared to the combined container-syringes 1, 100, and 110 having the finger contact cover 49, unevenness and step portions (the unevenness of the finger contact cover 49 itself, step portions formed between the front end of the finger contact cover 49 and the side surface of the safety device body 51, and the like) are reduced, thereby enhancing operability.

In addition, the coil spring 59 accommodated in the safety device body 51 is completely covered by the finger contact cover 140 having the cylindrical shape. Therefore, for example, as the finger contact cover 140 is composed of an opaque member, in use of the combined container-syringe 120, the coil spring 59 that has a tendency incite anxiety in patients is hidden by the finger contact cover 140 and is not viewed by the patients. As a result, in use of the combined container-syringe 120, unnecessary anxiety is not given to the patients. In addition, the finger contact cover 140 may be colored, and the color may be changed depending on the kind of the medicine sealed in the combined container-syringe 120. In this case, the kind of the medicine sealed in the combined container-syringe 120 may be easily identified from the color of the finger contact cover 140, so that convenience of the combined container-syringe 120 is enhanced.

On the other hand, in the combined container-syringe 120, the diameter of the front end portion of the safety device 50 is slightly reduced. Specifically, in the safety device 50, the inside diameter of a part covering the periphery of the luer lock portion 34 and the needle base 39 is reduced to be fitted to the outside diameter of the luer lock portion 34. In this case, the part with the reduced diameter is formed by fitting, to the front end of the safety device body 51, a cylindrical cap 150 in which the rear side has substantially the same diameter as that of the safety device body 51 and the diameter of the front side is reduced to be fitted to the luer lock portion 34.

By reducing the diameter of the front end of the safety device 50, compared to the first to third embodiments in which the front end of the safety device 50 has substantially the same diameter as the rear end side thereof, the front end of the safety device 50 is less likely to touch the skin of a patient in use of the combined container-syringe 120. As a result, an injecting operation becomes easy, and operability of the combined container-syringe 120 is enhanced.
In addition, as illustrated in FIG. 23, after the operation of the safety device, the front end of the injection needle 37 is covered by the front end of the advancing safety device 50. However, in the case of this embodiment, the diameter of the front end of the safety device 50 is reduced. Therefore, a finger or the like does not enter the inside of the safety device 50 via the front end of the safety device 50, and safety of the combined container-syringe 120 is further enhanced.
In addition, in the case where the diameter of the front end of the safety device 50 is reduced, the cap 150 in which the diameter of the front side is reduced may be fitted to the front end of the safety device body 51, or the diameter of the front end portion of the safety device body 51 may be reduced in the same shape as the cap 150.

While the embodiments of the combined container-syringe of the invention have been described in detail, the invention is not limited to these embodiments as long as the embodiments do not depart from the technical concept, and slight design changes may be made.

For example, the safety device 50 is composed of the safety device body 51 and the pressing ring 60, and the return prevention stopper 54 protrudes or retreats according to the position of the pressing ring 60. However, the configuration of the safety device 50 is not limited to this, and may be a configuration in which the return prevention stopper 54 protrudes from the outset. In addition, in this case, it is preferable that the height of the slip-out prevention stopper 53 is higher than the height of the return prevention stopper 54. Accordingly, when the safety device 50 is mounted from the tip end side of the main body of combined container-syringe 10, it may be easily mounted, and when the injection needle 37 is covered by the safety device 50, the safety device 50 is reliably fixed, thereby preventing the safety device 50 from deviating from the main body of combined container-syringe 10.

In addition, in the embodiment, although the coil spring 59 is disposed between the rear end portion of the safety device 50 and the finger grip 40 of the combined container-syringe 10, the coil spring 59 may be configured to be disposed on the tip end side of the safety device 50. Furthermore, in this embodiment, the invention is applied to a so-called two-chamber type combined container-syringe in which medicine and medical solution are respectively sealed in spaces formed between a front stopper and a middle stopper and between the middle stopper and a rear stopper, and the invention may also be applied to a so-called one-chamber type combined container-syringe in which medical solution is sealed in a space formed between the front stopper and the rear stopper.

### Industrial Applicability

According to the invention, in the combined container-syringe having the safety device used for ensuring safety by covering the injection needle after an administration of injection, the safety device may be easily moved to a position where the injection needle is covered. In addition, a malfunction of the safety device during the injecting operation may be reliably prevented.

### Reference Signs List

1, 100, 110, 120: combined container-syringe
3: first holding means (holding means)
10: main body of combined container-syringe
20: outer cylinder
30: hub luer-lock (cylindrical tip)
37: injection needle
40: finger grip
44: fastening convex portion (fastening portion)
49, 140: finger contact cover
50: safety device
58: engagement plate (engagement portion)
58c: rib
59: coil spring (biasing means)
70, 90: locking means
6, 93: pressing portion
80, 91: fastening releasing means

## Claims

1. A combined container-syringe comprising:
a container-cum-syringe body in which a cylindrical tip to which an injection needle is mounted is attached to a front end side of an outer cylinder that is filled with a medical solution and extends along an axis, and a finger grip is attached to a rear end side of the outer cylinder;
a safety device which is slidably mounted to an outer peripheral side of the outer cylinder, and covers the injection needle when being moved forward;
biasing means for biasing the safety device forward;
holding means for causing the safety device to be in a fastened state at a retreated position against biasing of the biasing means;
fastening releasing means which is provided slidably along a radial direction of the axis and releases the fastened state of the safety device by the holding means according to a sliding movement; and
locking means for locking the fastened state of the safety device by the holding means.

2. The combined container-syringe according to claim 1, wherein the fastening releasing means is configured to, during the sliding movement from a standby position positioned on an outside in the radial direction to a release position positioned on an inside in the radial direction, first, release the locking by the locking means, and second, release the fastening by the fastening releasing means.

3. The combined container-syringe according to claim 1,
wherein the holding means includes a fastening portion provided in the main body of combined container-syringe, and an engagement portion that is provided at a rear end portion of the safety device and is engaged with the fastening portion from the outside in the radial direction, and
the locking means has a pressing portion that presses the engagement portion from the outside in the radial direction.

4. The combined container-syringe according to claim 3, further comprising a rib on which the pressing portion abuts, on a surface on the outside in the radial direction of the engagement portion.

5. The combined container-syringe according to claim 1,
wherein the locking means is provided rotatably with respect to the main body of combined container-syringe, and
the fastening releasing means abuts on the locking means when the fastening releasing means is slid, and as the locking means is rotated, the locking by the locking means is released.

6. The combined container-syringe according to claim 1,
wherein the locking means is provided slidably along the radial direction, and
as the locking means is slid as the fastening releasing means is slid, the locking by the locking means is released.

7. The combined container-syringe according to any one of claims 1 to 6, wherein, in the finger grip, a finger contact cover that surrounds an outer peripheral portion of a rear end of the safety device at the retreated position from an outer peripheral side is provided.
